(19) Europäisches Patentamt / European Patent Office / Office européen des brevets

(11) **EP 3 921 643 B1**

(12) **EUROPEAN PATENT SPECIFICATION**

(45) Date of publication and mention of the grant of the patent:
**14.02.2024 Bulletin 2024/07**

(21) Application number: **19809630.7**

(22) Date of filing: **19.11.2019**

(51) International Patent Classification (IPC):
**G01N 33/543** *(2006.01)*     **G01N 27/327** *(2006.01)*
**G01N 15/00** *(2024.01)*     **G01N 15/06** *(2024.01)*

(52) Cooperative Patent Classification (CPC):
**G01N 33/5438; G01N 15/0606; G01N 15/0656; G01N 27/3277;** G01N 15/01

(86) International application number:
**PCT/IB2019/059922**

(87) International publication number:
**WO 2020/104933 (28.05.2020 Gazette 2020/22)**

(54) **INTEGRATED TEST CARTRIDGE FOR MEASURING ANALYTE CONCENTRATION**

INTEGRIERTE TESTKARTUSCHE ZUR BESTIMMUNG DER ANALYTKONZENTRATION

CARTOUCHE DE TEST INTÉGRÉE POUR MESURER LA CONCENTRATION D'ANALYTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2018 US 201862769038 P**

(43) Date of publication of application:
**15.12.2021 Bulletin 2021/50**

(73) Proprietor: **Zio Health Ltd.**
**London, SW8 2BW (GB)**

(72) Inventors:
• **LIU, Yu**
  **Harrow, Middlesex HA1 2AX (GB)**
• **RYAN, Rory**
  **Harrow, Middlesex HA1 2AX (GB)**
• **LIANG, Shaolin**
  **Harrow, Middlesex HA1 2AX (GB)**

(74) Representative: **Baldwin, Mark**
**Firebird IP**
**27 Old Gloucester Street**
**London WC1N 3AX (GB)**

(56) References cited:
WO-A1-2015/155665     WO-A1-2018/002693
WO-A2-2015/001050     US-A1- 2014 072 959
US-A1- 2015 068 922     US-A1- 2016 166 186
US-A1- 2017 014 822     US-B2- 9 310 363

• JAMES S. SWENSEN ET AL: "Continuous, Real-Time Monitoring of Cocaine in Undiluted Blood Serum via a Microfluidic, Electrochemical Aptamer-Based Sensor", JOURNAL OF THE AMERICAN CHEMICAL SOCIETY, vol. 131, no. 12, 1 April 2009 (2009-04-01), pages 4262-4266, XP055036845, ISSN: 0002-7863, DOI: 10.1021/ja806531z

• HUI LI ET AL: "Calibration-Free Electrochemical Biosensors Supporting Accurate Molecular Measurements Directly in Undiluted Whole Blood", JOURNAL OF THE AMERICAN CHEMICAL SOCIETY, vol. 139, no. 32, 16 August 2017 (2017-08-16), pages 11207-11213, XP055663955, ISSN: 0002-7863, DOI: 10.1021/jacs.7b05412

• SÁNCHEZ J L ACERO ET AL: "Multiplex PCB-based electrochemical detection of cancer biomarkers using MLPA-barcode approach", BIOSENSORS AND BIOELECTRONICS, ELSEVIER SCIENCE LTD. UK, AMSTERDAM, NL, vol. 82, 7 April 2016 (2016-04-07), pages 224-232, XP029527875, ISSN: 0956-5663, DOI: 10.1016/J.BIOS.2016.04.018

• YING LIU ET AL: "Aptamer-Based Electrochemical Biosensor for Interferon Gamma Detection", ANALYTICAL CHEMISTRY, vol. 82, no. 19, 1 October 2010 (2010-10-01), pages 8131-8136, XP055683803, US ISSN: 0003-2700, DOI: 10.1021/ac101409t

- DANIEL EVANS ET AL: "A Novel Microfluidic Point-of-Care Biosensor System on Printed Circuit Board for Cytokine Detection", SENSORS, vol. 18, no. 11, 17 November 2018 (2018-11-17), page 4011, XP055683729, DOI: 10.3390/s18114011
- HADI MIRZAJANI ET AL: "A highly sensitive and specific capacitive aptasensor for rapid and label-free trace analysis of Bisphenol A (BPA) in canned foods", BIOSENSORS AND BIOELECTRONICS, vol. 89, 1 March 2017 (2017-03-01), pages 1059-1067, XP055683763, AMSTERDAM, NL ISSN: 0956-5663, DOI: 10.1016/j.bios.2016.09.109
- LEI WANG ET AL: "An Electrochemical Aptasensor Using Coaxial Capillary with Magnetic Nanoparticle, Urease Catalysis and PCB Electrode for Rapid and Sensitive Detection of Escherichia coli O157:H7", NANOTHERANOSTICS, vol. 1, no. 4, 1 January 2017 (2017-01-01) , pages 403-414, XP055683760, ISSN: 2206-7418, DOI: 10.7150/ntno.22079
- FELISMINA T.C. MOREIRA ET AL: "Screen-printed electrode produced by printed-circuit board technology. Application to cancer biomarker detection by means of plastic antibody as sensing material", SENSORS AND ACTUATORS B: CHEMICAL, vol. 223, 1 February 2016 (2016-02-01), pages 927-935, XP055683726, NL ISSN: 0925-4005, DOI: 10.1016/j.snb.2015.09.157

**Description**

FIELD OF THE INVENTION

The invention lies in the field of biosensors, specifically electrochemical aptasensors.

BACKGROUND

A biosensor, as used herein, refers to an analytical device that converts the detection of a chemical substance into a quantifiable signal. The chemical substance of interest will be referred to herein as an 'analyte'. Briefly, the main components of a biosensor are the bioreceptor that recognizes the analyte, the substrate upon which the interaction takes place and gives rise to a signal, and the transducer that quantifies the interaction in some physicochemical way. An extremely wide variety of biosensors have been used and described at the time of this writing, with variations in biological principles, transduction methods, manufacturing and design, across all three main components. Most existing devices are limited to research and clinical use, as they are expensive to manufacture and require specialized training and knowledge to use.

Today, antibodies are the most commonly used natural bioreceptor across the industries. Antibodies have specific binding affinity for their antigen, in which the antibody and the antigen will only bind if they have the correct conformations. They are commonly used in conjunction with fluorescent or colorimetric optical transduction.

Aptamers are oligonucleotides or peptide molecules that bind to a specific target molecule. Like antibodies, they bind with high affinity to target molecules based on three-dimensional conformations that interact with the complementary target molecules. Upon binding, some aptamers undergo conformational change. Colourimetric platforms are popular in academic environments, but as of yet there is no dominant design in the industry, in part because there are no or very few commercial devices utilising aptamers in diagnostic devices.

While optical transduction is a common method for transduction, electrochemical transduction is also growing in use. Electrochemical biosensors are those that transduct the detection of analyte into current or voltage. In electrochemical biosensors, the substrate typically comprises three electrodes: reference, counter and working. Voltage is applied across the counter and working electrode, where the analyte recognition interaction takes place.

Currently, a variety of biosensor electrodes are available by existing manufacturing methods, the most commonly available being the disk electrode. The disk electrode comprises a polyetheretherketone (PEEK) tube that contains a thin layer of conductive metal, but it is not suitable for low-cost commercial use, only for research purposes. Disk electrodes require the separate preparation of working, reference and counter electrodes, and there is little control over the thickness of the substrate layer.

Sputtering and magnetron sputtering are more sophisticated manufacturing techniques, using thin film deposit processes that require very expensive equipment and thus for biosensing applications are not suitable for mass production, only for research purposes. The equipment requires a vacuum to operate, typically nitrogen is used to fill and clean the substrate surface, and the equipment is physically large. Pre-treatment must be applied to the biosensor substrate before the electrode material can be applied to the substrate. The connection between substrate and electrode material is not strong. The connection typically is not a chemical bond, it relies upon physical adsorption. Therefore, the electrode material has only a weak bond to the substrate and so can be easily removed from the substrate and the electrode surface can easily be scratched or destroyed during use.

For commercial applications, currently only screen-printed electrodes are readily available. The support material can be a soft or flexible material such as plastic. The performance is acceptable, but screen-printed electrodes tend to perform at a lower quality than disk electrodes and sputtering electrodes. The electrode material is not a solid material, rather it is a powder material and it is also mixed with an adhesive material or solvent, causing the conductivity of the electrode to be reduced as compared to a solid material. The electrode material powder is not a pure powder. Electrode material particles do not always connect with other electrode material particles, because adhesive particles fit in between, causing conductivity to be reduced. The rejection rate of screen-printed electrode during manufacturing processes is high. At the time of writing, leading commercial screen-printed electrodes such as Dropsens DS220AT and DSC223BT cost 405 USD for 75 electrodes and 365 USD for 75 electrodes, respectively, relatively expensive for integration into an affordable consumer product. In addition, the quality of the electrode is not suitable for biochemistry that requires modification to pure electrode material surface because the adhesive material mixed with the pure electrode material blocks the modification process.

The manufacture of printed circuit boards (PCBs) is used predominantly for the manufacturing of electronic circuits and there is an existing and large global infrastructure supporting this manufacturing method that is larger than the manufacturing infrastructure supporting screen printed biosensor electrode platforms and sputtering biosensor electrode platforms. However, to date only a few cases of use of PCBs to produce biosensor electrode platform have been attempted for research purposes, because high quality biosensor measurements have not been achievable with this manufacturing method. PCB electrodes show poorer signal than screen printed electrodes and are generally not thought to be suitable for use.

Most commercially available biosensors, to the extent they exist today, require microfluidics to control the sample reaction. This is because assay conditions at the sensor element such as temperature and pH, usage requirements such as the mixing of reagents and the volume of test sample, and signal interference issues at the electrodes, to name a few design concerns, must all be taken into account, and thus specific locations for each step must be carefully accounted for. The term "microfluidics" as used herein refers to the passive or active physical manipulation of sample to drive or direct the movement of fluid. Microfluidics therefore incorporates the use of mechanical, electromechanical, vacuum or piezoelectric pumps, or some other actuation method, to drive or direct sample to individual test zones, or to direct sample and/or reagent to a common locale. For instance, microfluidics may be implemented to facilitate mixing sample with a reagent or to move the sample from a sample collector at one location to a biosensor element to another location. Due to the sensitivity of assay conditions or risk of electronic interference at the electrodes, these locations may be quite separated on the device. For instance, the DropSens 8x110 comprises eight 3-electrode electrochemical cells, with separate reference and counter electrodes for each working electrode. Although it is disposable, this design is not suited to widespread commercial use because of the large size of the test strip. It requires the distribution of test sample into multiple test zones using multichannel micropipettes and checking that sufficient sample has been applied to each test zone to fully cover all electrodes in each test zone.

Another obstacle to widespread commercial biosensor applications is the problem of calibration of each sensor. Because the manufacturing of biosensors is imperfect and even slight variations in their fabrication can influence the resulting signal output from any given biosensor, accuracy of any given test for measuring target can only be ensured by calibration of each biosensor with a target-free, or "blank" test. Calibration performed before the point of use, such as during manufacturing, risks being invalidated once changes occur at the sensor due to distribution and shelf-life time. Even if this problem were to be solved, if calibration is performed during manufacturing, it significantly increases the costs of production of biosensor devices and makes it impossible to mass produce biosensors designed for single test disposable applications. Calibration further illustrates another reason for microfluidics. Even if a single test disposable application were to be designed, microfluidics would be needed to direct a blank sample to the biosensor, along with or just before the sample under test, at the point of use.

SANCHEZ J L ACERO ET AL, "Multiplex PCB-based electrochemical detection of cancer biomarkers using MLPA-barcode approach", BIOSENSORS AND BIOELECTRONICS, ELSEVIER SCIENCE LTD. UK, AMSTERDAM, NL, (20160407), vol. 82 discloses a PCB electrode for carrying out multiplexed electrochemical detection assays comprising an array of individually addressable working electrodes, a common reference electrode and a counter electrode. The electrodes are disposed on the top surface of the PCB. The PCB has edge connectors that slots into two interface PCBs. A Peltier block and heat sink are in engagement with the bottom surface of the PCB.

HADI MIRZAJANI ET AL, "A highly sensitive and specific capacitive aptasensor for rapid and label-free trace analysis of Bisphenol A (BPA) in canned foods", BIOSENSORS AND BIOELECTRONICS, AMSTERDAM, NL, (20170301), vol. 89, discloses a PCB electrode that has interdigitated electrodes that have connection points on the top surface of the PCB.

LEI WANG ET AL, "An Electrochemical Aptasensor Using Coaxial Capillary with Magnetic Nanoparticle, Urease Catalysis and PCB Electrode for Rapid and Sensitive Detection of Escherichia coli O157:H7", NANOTHERANOSTICS, (20170101), vol. 1, no. 4 discloses a PCB electrode that has interdigitated electrodes that have connection points on the top surface of the PCB.

SUMMARY

The invention provides a test strip for reporting a plurality of analyte concentrations of a sample as specified in claim 1. The invention also includes an integrated test cartridge as specified in claim 4.

Embodiments of the invention provide an accurate, mass-producible integrated test cartridge and test strip that are suitable for commercial use. The integrated test cartridge uses aptamers in a simple architecture that achieves accurate and reliable results. The test strip is designed to use existing PCB manufacturing technology while maintaining the accuracy of the biosensor function. There are no microfluidics required to facilitate biosensing within the integrated test cartridge, and no need for mixing with reagents. The user can put a drop of test sample directly into the integrated test cartridge and targets within the test sample will immediately diffuse and begin to bind with aptamers immobilised onto the electrode top surface. Signal is measured with electrochemical method. Furthermore, there is no additional calibration step required. Using a novel calibration method, the analyte concentration can be measured quickly and accurately at the point of use, without the need of consumer intervention and without the need for calibration with a separate control fluid. As a result, the integrated test cartridge is cheaper and easier to produce and distribute while maintaining high efficacy, as well as simple and intuitive to use at the consumer level.

Aptamer electrodes are printed on a single test strip using currently available PCB printing techniques to create the electrodes. The term "PCB electrode" as used throughout shall mean an electrode manufactured using PCB printing techniques. This PCB biosensor may be a multiplexing sensor for the detection of multiple aptamers. Each aptamer is modified with a redox-active molecule and selected so that in addition to high sensitivity, the aptamer changes the proximity of the redox-active molecule to the working electrode in the presence of the analyte, sending faradic discharge to the electrode surface. An existing electrochemical method, including, but not limited to, square-wave voltammetry (SWV), differential pulse voltammetry (DPV), linear sweep voltammetry (LSV), cyclic voltammetry (CV), electrochemical impedance spectroscopy (EIS), amperometry, and alternating-current (ACV), is used to convert the current to electric signal. Due to the high sensitivity of the aptamers, a plurality of working electrodes may test for a plurality of target analytes in a single test, without requiring multiple counter and reference electrodes or microfluidics to distribute sample to the working electrodes. By selecting the aptamers to be immobilized on the working electrodes, the platform is adaptable to provide specific tests relevant to the user.

The integrated test cartridge has been designed for intuitive and rapid use, as well as faster manufacturing. Rather than the horizontal construction demonstrated by existing biosensor cartridges, the test strip is printed with electrode connectors directly under the working surface of the test strip. The test strip can be quickly snapped into a test cartridge, speeding up the manufacturing process. Moreover, the test strip is located directly under a sample collector with an opening at its base whereby a fluid sample can simply flow downward over the test strip due to gravity. The need for microfluidics is thus eliminated. The test cartridge requires no reservoirs, reagents or mixing steps. Furthermore, the test cartridge is designed to attach vertically to a reader device. The vertical design of the test cartridge with test strip underneath, and the test cartridge attaching vertically to the reader device, ensures tight sealing without additional fasteners. This is crucial to avoid spillage and damage to the sensitive electronics and is also a simple and intuitive user interface.

The invention enables new biosensor devices in the form of test strips and integrated cartridges as specified in the claims to be manufactured at a cost cheaper than traditional screen-printed biosensor electrode platforms (typically a factor of 20 times less than existing screen-printed electrodes). Furthermore, the invention allows the utilization of an existing, large global infrastructure to produce biosensor platforms.

BRIEF DESCRIPTION OF THE DRAWINGS

FIG. 1 is a top perspective view of a test cartridge.
FIG. 2 is a bottom perspective view of the test cartridge.
FIG. 3 is a horizontal cross-sectional view of the test cartridge.
FIG 4 is a bottom plan view of the test cartridge.
FIG. 5 is a horizontal cross-sectional view of the test cartridge connected to the vertical connector of a reader device.
FIG. 6 is a horizontal cross-sectional view of a reader device.
FIG. 7 is a top perspective view of a reader device.
FIG. 8 is a schematic of a reader device with the test cartridge attached, in relation to a remote server and a user device.
FIG. 9 is a top perspective view of a test strip.
FIG. 10 is a bottom perspective view of the test strip.
FIG. 11A is a top plan view of the test strip.
FIG. 11B is a bottom plan view of the test strip.

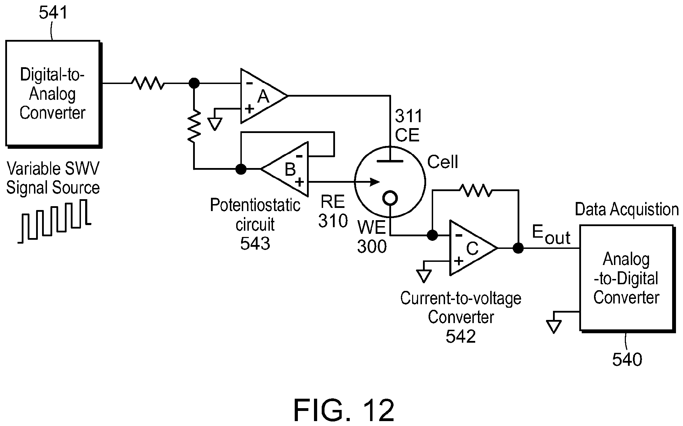

FIG. 12 is a schematic of a potentiostatic circuit for operating the test strip.
FIG. 13 is a schematic visualization of aptamer binding with target and aptamer conformational folding with target resulting in large discharge current.
FIG. 14 is a cross-sectional view of a working electrode showing a conductive layer, aptamer layer and barrier layer.
FIG. 15 is a graphical visualization of a square wave voltammetry signal resulting from aptamer conformational change.
FIG. 16 is a graph plotting $\gamma_1 = i_{max1}/i_{min1}$, $\gamma_2 = i_{max2}/i_{min2}$, and $z = i_{min1}/i_{min2}$, which are shown to be constant across multiple test strips.

DETAILED DESCRIPTION

I. <u>Test Cartridge Architecture</u>

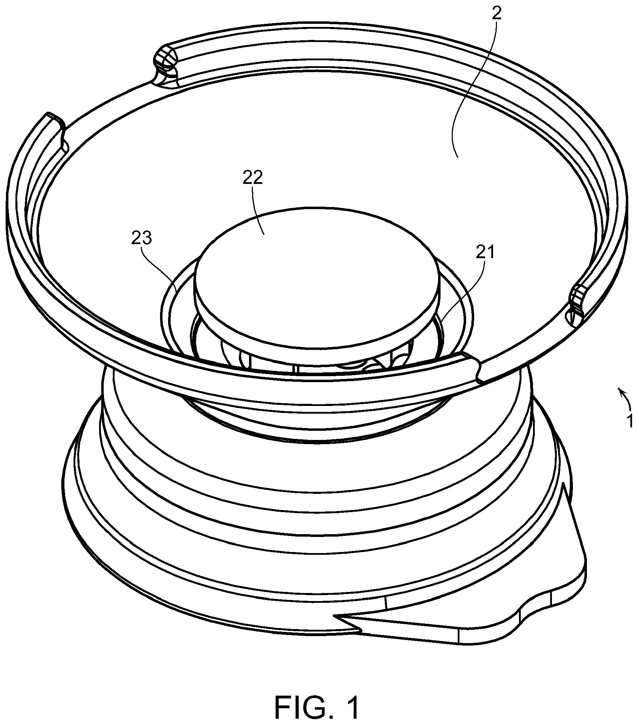

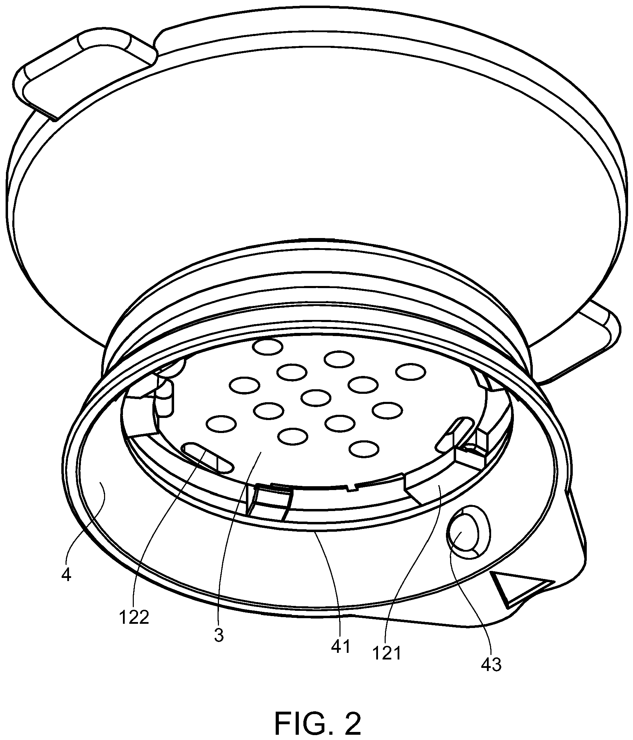

Referring to Figs. 1 to 3, an integrated test cartridge 1 for accepting and analyzing a fluid sample for the presence of one or more analytescomprises a sample collector 2 that accepts a test strip 3 directly under the sample collector 2 and furthermore attaches vertically to a reading device 5 comprising circuit board 54, operable to cause the test strip 3 to be activated and to transmit resulting measurements of analyte concentrations. The integrated test cartridge 1 eliminates the need for any microfluidics design, does away with the use of fasteners that increase manufacturing and labor costs and risks of wear and malfunction, and requires no additional reservoirs for calibration fluids or mixing reagents. Rather, the fluid sample simply flows downward through an opening 21 in the base of the sample collector 2 and over the test strip 3 for testing. As such, as will be used herein, the phrase "direct fluid connection" means without the use of microfluidics to direct the sample, i.e., pursuant to the description and definition of microfluidics in paragraph 0011, without additional structures to drive or direct the movement of fluid.

The sample collector 2 accepts the sample, which flows downwards over the surface of the test strip 3, which is in direct fluid connection with the sample collector 2. The shape of the sample collector 2 shown in the drawings is in the shape of a cone. However, the sample collector may come in a variety of shapes and sizes without departing from the scope of the invention as specified in the claims. Different sample collectors may be designed for all types of fluid samples, such as blood, saliva, or even non-bodily fluids such as infant formula. For a sample such as breast milk, a cone shaped collector may be preferable because it allows the user to pour the breast milk from bottle into the sample collector. The integrated test cartridge 1 may comprise a visible volume indicator 23 within the sample collector 2 to indicate to the user the required volume of fluid sample they must drop into the cartridge. The sample collector 2 may comprise a filter 22 and/or other pre-treatment mechanisms such as active charcoal carbon filters, which may serve to remove materials that have a high risk of causing false positives and false negative test results. The sample collector 2 may be constructed of any material, or of several parts and materials, including durable materials that can be washed and reused, so that for instance, the test strip can be disposed of after use and a new test strip can be reinserted into the test cartridge, thereby reducing plastic waste).

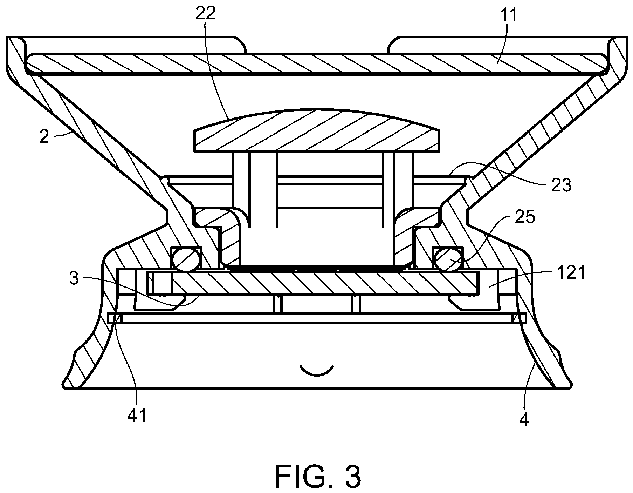

It is preferable that cartridge 1 also comprises a lid 11 (Fig. 3) to cover the sample collector 2 which ensures a closed system. Ensuring that the test strip 3 operates in a closed system while the reader device 5 analyses the sample is important to prevent external contaminants entering the cartridge and reacting with the test strip, resulting in greater noise. The lid 11 also preserves the test strip biochemistry shelf-life during storage and distribution of cartridges and also prevents the fluid sample from spilling while the reader device analyses the sample and during cartridge disposal. The lid 11 may be a separate component or formed of one piece with the sample collector with a living hinge.

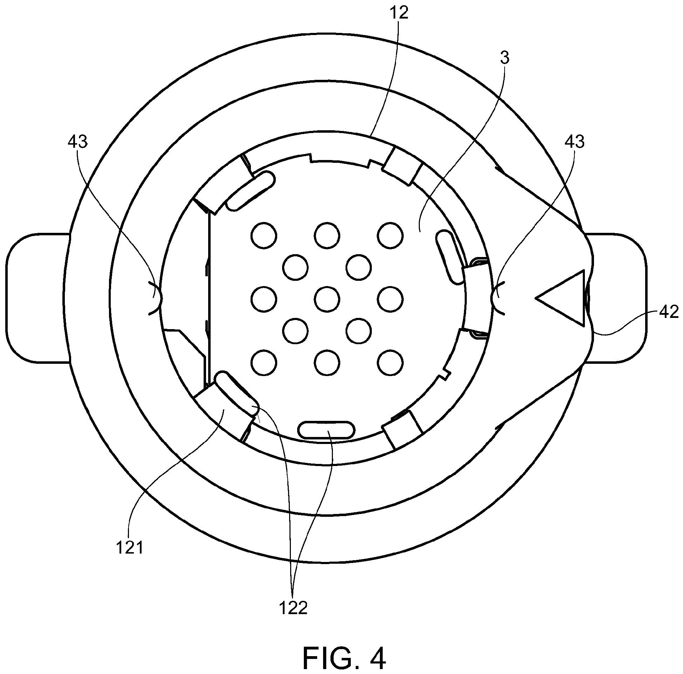

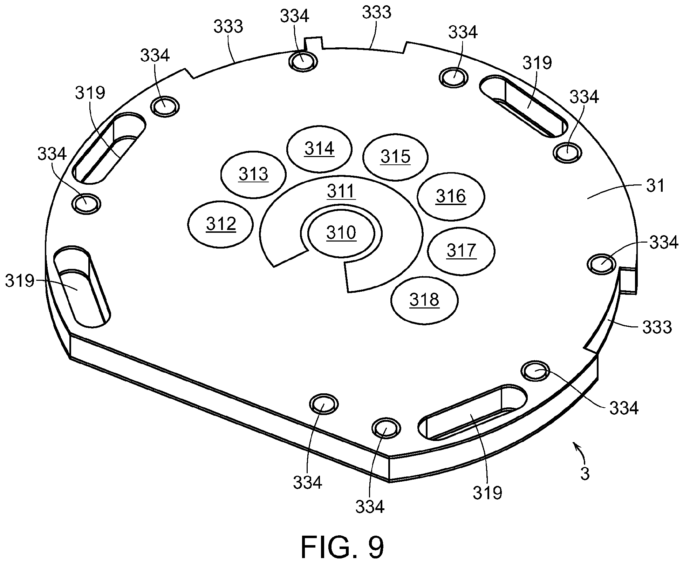

Referring to Fig. 3, the test strip 3 is fitted to the underside of the sample collector 2 so as to be in fluid connection with the sample collector 2 via opening 21. The cartridge is designed to accept the test strip 3 at a test strip interface 12 (Fig. 4) located at the base of the sample collector 2. The test strip interface 12 may be specifically designed to fit the form of the sample collector and/or the test strip. In the embodiment depicted, the test strip interface 12 is comprised of an O-ring 25 and a series of test strip insertion clips 121 below the O-ring 25 operable to secure test strip 3 against the O-ring so that a watertight seal is formed. The test strip 3 is comprised of cutaways 333 (Fig. 9) that correspond to the insertion clips 121. The test strip 3 can be inserted into the test strip interface 12 using a simple and fast screw mechanism whereby the test strip is inserted into the test strip interface 12 and twisted until secured in place by the insertion clips. This rotational step applies sufficient force to secure the test strip tightly against the O-ring 25, or equivalent watertight seal. No fasteners or glue are required, instead pressure is applied to the test strip via insertion clips 121. The advantage of this design is that it reduces the need for additional materials or cumbersome manual assembly, therefore reducing the overall manufacturing cost of the cartridge. Notably, the entire cartridge 1, including all parts such as test strip interface 12, device connector interface 4 (Fig. 4), sample collector 2 and lid 11 may be produced from a single piece of material, significantly reducing risks of wear and tear on parts and the cost of assembly. The embodiment depicted in the drawings depicts a disc-shaped test strip, which corresponds to the technique of using test strip insertion clips 121 to twist into place against the O-ring 25. However, it is possible to design the tes- strip 3 to be a different shape, with corresponding alterations to the test strip interface 12, without departing from the scope of the claims.

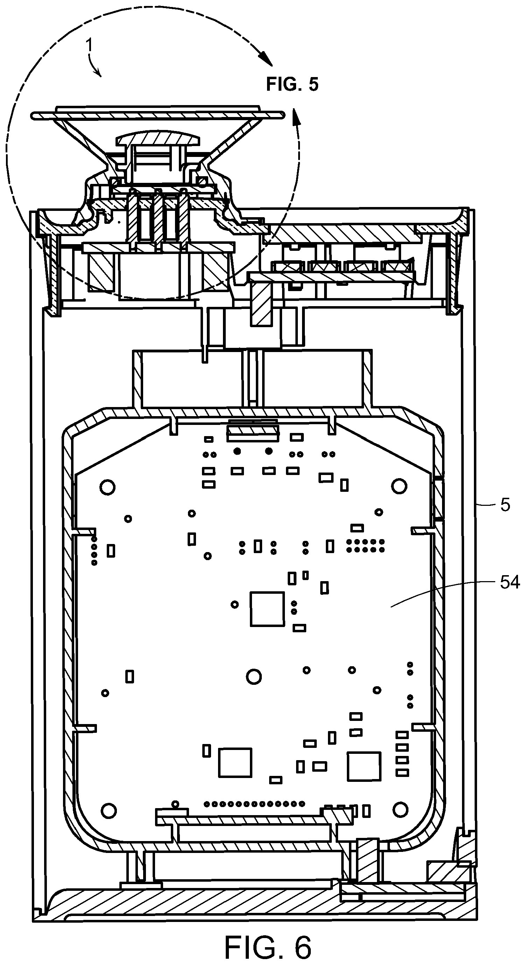

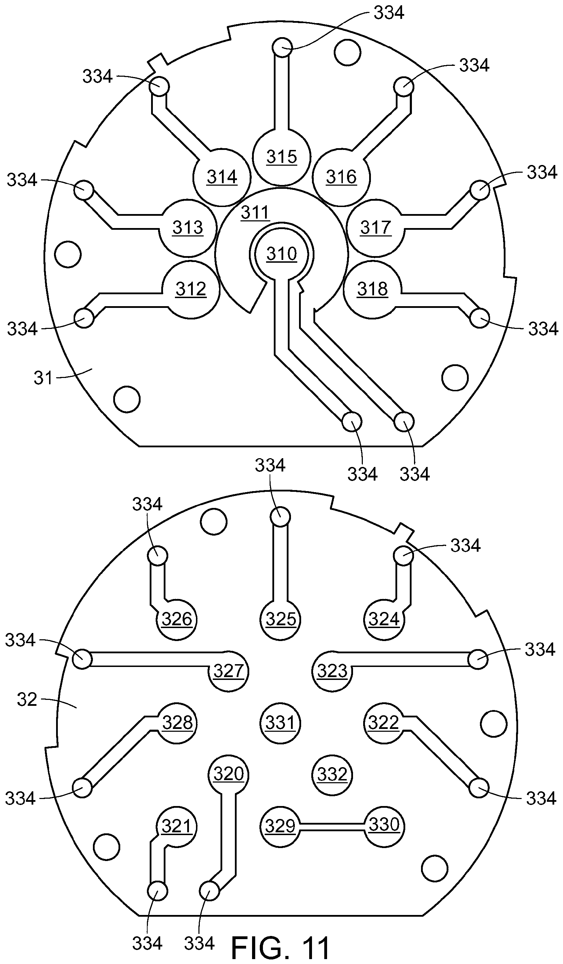

The PCB printing technique and electrode design on the test strip (discussed further in Section II) allows the integrated test cartridge 1 to be connected to the top of the reader device 5 vertical connector 51 in a vertical fashion (as shown in Fig. 6) rather than inserted in a horizontal fashion into an analyser as is the current state of the art. As further discussed in Section II, the novel test strip design facilitates this vertical connection. Referring to Figs. 9 to 11, on the bottom surface 32 of the test strip 3, an arrangement of contact points 320-331 (Fig. 11) is in operable connection with the electrodes 310-318 on the top surface 31 through PCB vias. Conductive pins 52 on the vertical connector 51 and operably connected to the circuit board 54 of the reader device 5 touch the contact points 320-331 when a cartridge 1 is inserted onto a vertical connector 51.

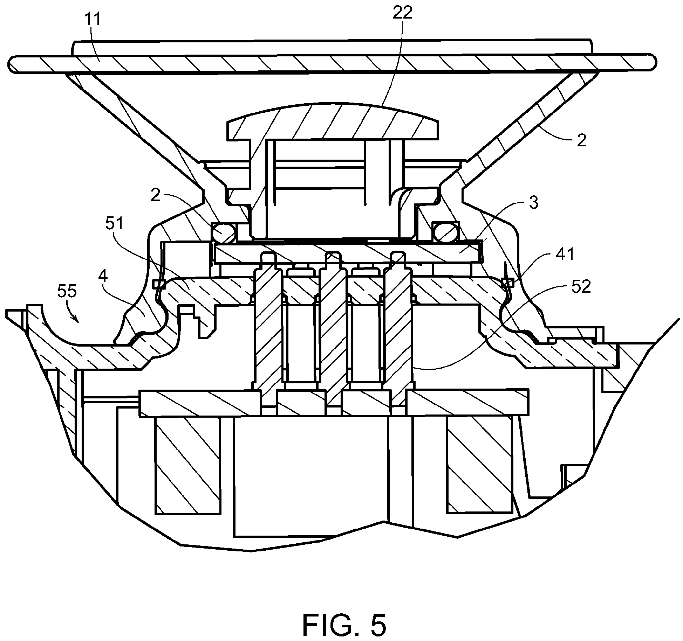

A vertical connector allows the junction between the integrated test cartridge 1 and reader device 5 vertical connector 51 to be located directly below the bottom surface 32 of the test strip 3 resulting in a much more compact design. A second, watertight seal 41 (Fig. 5) seals the integrated test cartridge 1 to the vertical connector, preventing leakage onto the pins 52, which can result in short circuit, device damage and other errors. Because the conductive wire transducers between the electrodes 310-318 and the contact points 320-331 on the bottom surface of the test strip 3 do not run in parallel as in a horizontal connector design, cross-talk interference from inductive coupling of wires during high frequency or high current measurement signals is reduced and signal quality is improved. While multiplexing biosensors with large numbers of working electrodes necessitate wider horizontal connectors to house all of the electrodes, the vertical connector allows pins to be arranged in a 2-dimensional plane (rather than along one parallel edge on a horizontal connector) such that more pins can be fitted in the same area.

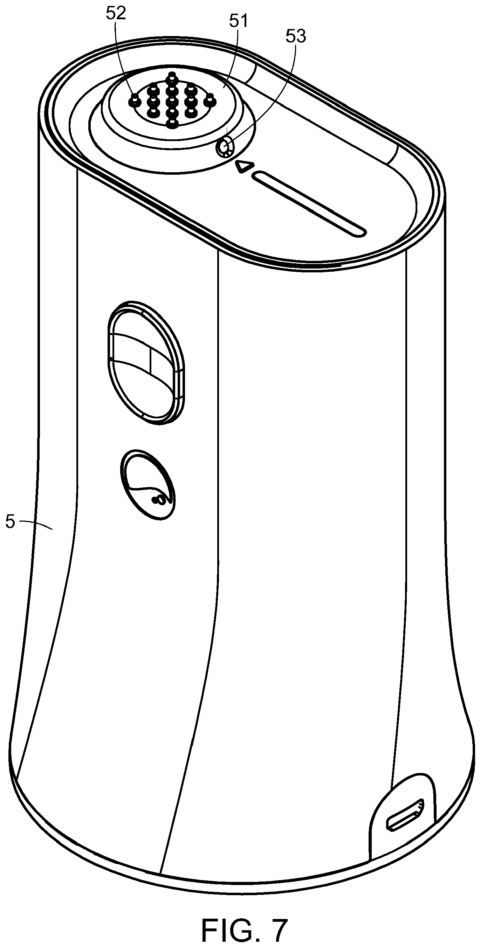

The integrated test cartridge 1 may be mechanically locked onto the vertical connector 51 to prevent a cartridge from disconnecting from reader device 5 during analysis. Specifically, the cartridge 1 comprises a reader device interface 4 surrounding the test strip interface 12 and located under the sample collector 2, thus facilitating the vertical connection to the reader device 5. The reader device interface 4 may be comprised of a snap in connector 43 that snaps over the top of the vertical connector 51, but other connectors that would be known in the art, such as threads or magnets, may be used without departing from the scope of the claims. The reader device interface 4 surrounds the vertical connector 51 thereby preventing contamination and/or accidental spillage of sample onto the reader device 5 and/or its vertical connector 51, which in turn prevents electrical short-circuit noise during analysis. Preferably the reader device interface 4 also comprises a second watertight seal 41 to prevent sample leakage into the cartridge connector. The form of the reader device 5 may comprise a reservoir 55 (Figs. 5 to 7) at the top of the device to help the user easily collect excess fluid sample that that user may have spilt onto the reader device during the deposit of fluid sample into the cartridge. The design of the cartridge may comprise a sealed film or fuse that can be broken by the user before use but not repaired to act as an indicator that the cartridge has been used already before and should not be used again. The design of the reader device interface 4 may comprise visible cartridge orientation indicators 42 to indicate to the user the correct orientation to align the reader device interface 4 to enable proper connection to the vertical connector 51 pins 52.

The form of the cartridge 1 allows multiple cartridges to be easily stacked on top of each other, thus reducing the overall packing size of multiple cartridges stored together during storage and distribution. This allows for reduced storage space required to store cartridges and reduced distribution costs for cartridges. In some applications, the reader device contains a storage magazine of multiple stacked cartridges and/or multiple stacked test strips within it. An automatic mechanism within the reader device retrieves a new cartridge or biosensor platform from the storage magazine and places it on the reader device vertical connector. After the user has placed fluid sample into the cartridge, run the test and received test results, the automatic mechanism retrieves the used cartridge or test strip and disposes of it within a waste storage magazine within the reader device. A user can insert new storage magazines containing new cartridges or test strips within the reader device and dispose of the waste storage magazine more infrequently than inserting and disposing of individual cartridges, which is a benefit to the user experience, especially in time critical applications.

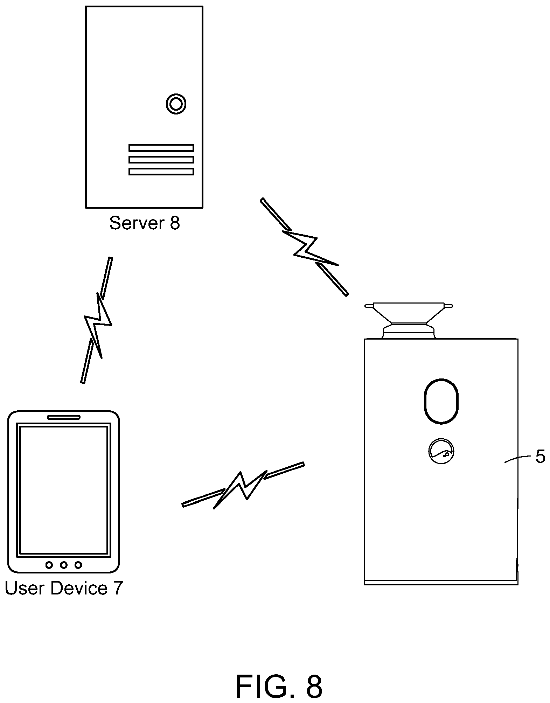

Each cartridge, in the case of disposable cartridges, or test strip, in the case of disposable test strips, is associated with a unique identification code through which a computing device may identify test settings or other information associated with the cartridge. The information may include the expiry date of the cartridge and the precise constants used to calculate the analyte concentration accurately for this manufacture (which will be discussed further in Section III below). The identification code may be affixed to or stored on the cartridge or test strip in the form of a Radio-Frequency Identification chip (RFID chip), a QR Code, serial number EEPROM chip or MAC address chip or arrangement of resistors or other passive components to form a uniquely identifiable code specific for each cartridge. A computing device, such as a smartphone 7 (Fig. 8) or even the reader device 5, may be programed with the ability to read this cartridge identification, which can be matched against a database of identification codes that is stored either locally or on a remote server. This allows for proper authentication of each cartridge before or after a user conducts a fluid sample test with the cartridge and allows for supply chain management of cartridges. The code further enables the application of the analyte calculation method, such as the Self-Parative Calibration Method, which is further discussed in Section III below.

The reader device 5 may also comprise features such as a cartridge locking mechanism and cartridge detector to detect the insertion of the cartridge. Bluetooth or Wifi protocol or other wireless or wired communication protocol may be used to connect the reader device 5 to a remote server 8 to analyze, store and manage test result data, or to connect the reader device to a personal computing device 7. The reader device 5 may also comprise a user interface to show information about testing status, including, but not limited to LEDs, LED array, LCD screen or other interfaces. In cases where a personal computing device 7 is used with the reader device 5, authentication of the device occurs during initial connection of the reader device and personal computing device. A pairing process occurs whereby unique identifier of each device is recorded in the reader device and the personal computing device and checked as an authentication method when connecting to the respective device in the future.

The reader device 5 in some forms maybe be embedded into other materials and objects and products such that the user experience is improved or facilitates more frequent testing. Some examples include embedding the reader into a personal electronic computer or device protection case or cover, a watch or wristband, a purse or bag or keychain, a food or drinking fluid container such a baby milk bottle or liquid food thermos-flask, a baby powder preparation machine, a cooking utensil or appliance such as a cooking pot or frying pan, cutlery and crockery and such as a bowl or plate for human or animal use, a toilet bowl for urine sampling, a bath or shower or washing basin, or an alcohol breathalyzer tool used by police or security services.

II. Test Strip

The test strip 3 is designed to ensure high quality measurements by electrodes produced using PCB manufacturing methods. Furthermore, it is designed to receive and measure the analyte concentration of a sample in direct fluid connection with the top surface 31 of the test strip 3, i.e. without mixing of additional reagents, or localization over specific electrodes using microfluidics or the like. As it has previously been defined in paragraph 0034, the phrase "direct fluid connection" means without the use of microfluidics to direct the sample, i.e., pursuant to the description and definition of microfluidics in paragraph 0011, without additional structures to drive or direct the movement of fluid.

The test strip 3 is designed to connect vertically to a reader device 5 comprising a circuit board 54, such that the test strip 3 stands as the sole interface between a sample collector (such as the sample collector 2 of the preferred embodiment) and the pins 52 of a reader device 5, which are operable to activate and receive signal output from the electrodes of the test strip. This leads to a more compact device overall, and one that is more efficient to manufacture as well as to use, in addition to other advantages that are discussed throughout.

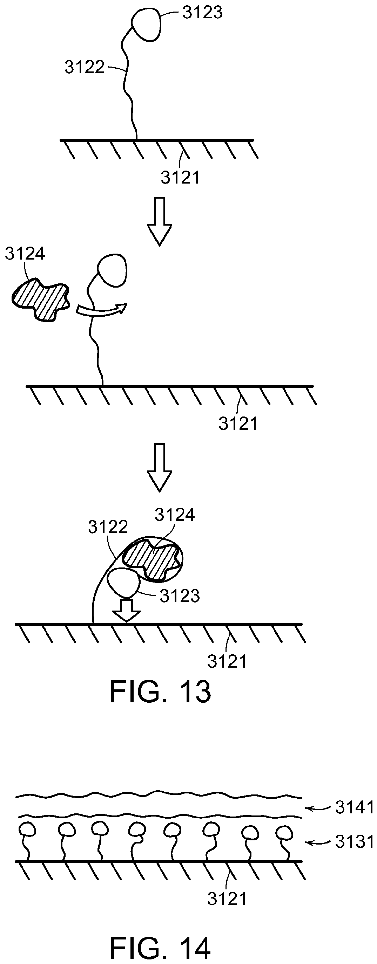

The test strip 3 comprises a top surface 31 and a bottom surface 32. On the top surface 31 are electrodes 310 - 318 printed on PCB substrate by chemical deposit of the conductive material with a modified surface layer forming the conductive layer 3121, on which is bound the aptamers 3122 corresponding to target molecule 3124 (Fig. 13). Due to their production by PCB printing process, these electrodes will be referred to as "PCB electrodes" throughout. Materials and combinations of materials such as, but not limited to gold, silver, platinum, copper, nickel, carbon, conducting polymer, metal particle, and other conductive materials can be used as the conductive layer 3121 upon which biochemistry can be modified. Electroless Nickel Immersion Gold surface plating is the preferred method for layering these materials over the PCB surface, where gold is the top surface layer for aptamer binding.

The aptamers are immobilized on the conductive layer 3121 by covalent bonding the aptamer to the gold surface layer with an active group such as thiol (-SH), carboxyl (-COOH), hydroxyl (-OH) at end (5' or 3'), thus forming the aptamer layer 3131. Modification of the working electrode surface can be achieved via biochemical coupling reaction such as NHS/EDC primary amine group modification or thiol-gold modification based on different materials of the electrode. The unbonded distal end of the aptamers 3122 is labeled with a redox-active molecule 3123 such as methylene blue (MB) or ferrocene (Fc). An aptamer with a redox-active molecule will change the proximity of the molecule to the conductive layer depending upon whether it is in a bound state or unbound state. In the example shown in FIG. 13, the aptamer 3122 folds towards the conductive layer 3121, but in different embodiments the aptamer may open away from the conductive layer or undergo different conformational changes. The conformational change results in greater faradic discharge from the redox-active molecule to the conductive layer 3121 if the redox molecule is brought in closer proximity to the conductive layer 3121, which can be sampled by the transduction method.

Aptamers 3122 must be designed to exhibit a conformational change when the aptamer strand binds to the target analyte. Aptamer may be comprised of deoxyribonucleic acid (DNA), ribose nucleic acid (RNA), nucleic acid analogues (XNA) and peptide. A blocking agent such as L-cysteine, 6-Mercapto-1-hexanol can be used to back-fill the space between aptamers to produce a more uniform distribution of aptamers and prevent nonspecific surface absorption of molecules in the test sample to the conductive layer 3121, resulting in slightly larger signal 'background noise'. Examples of suitable aptamers for adenosine triphosphate and tobramycin are found in the literature at Liu et al., "Achieving Reproducible Performance of Electrochemical, Folding Aptamer-Based Sensors on Microelectrodes: Challenges and Prospects," Analytical Chemistry 2014, 86, 11417-11424.

The aptamers for target analyte may be developed using SELEX (Systematic Evolution of Ligands by Exponential Enrichment) method, where they are selected for high binding affinity and conformational change of the aptamer whereby it folds (or opens wider) after binding to target. The aptamer sequence may be further modified to produce a greater structure change by: 1) truncating the aptamer to make it shorter by conducting experiments to approximate the region on the aptamer that binds to target and then removing DNA bases that are not in the binding region; and 2) mismatching bases in the aptamer's structure to make it less stable whereby when the aptamer binds to target the conformational change is more significant. The process of developing the aptamers may require several rounds of testing with the target to ascertain whether the aptamer shows signal change due to structure change.

As a result of the chemical deposit, the aptamer layer 3131 contains a large number of aptamers with methylene blue molecules, and the more target present in proximity to the electrode surface, the more aptamers bind and fold and the larger the average measured signal (faradic discharge current) across the conductive layer 3121 when potential is applied across each working electrode 312-318 and the counter electrode 311. The transduction of the resulting altered charge transfer rate may be performed by electrochemical methods such as square wave voltammetry (SWV), differential pulse voltammetry (DPV), linear sweep voltammetry (LSV), electrochemical impedance spectroscopy (EIS), amperometry, cyclic voltammetry (CV), or alternating-current (ACV). The first two methods are particularly well suited for monitoring surface-confined reactions, as they reduce non-faradaic background charging currents.

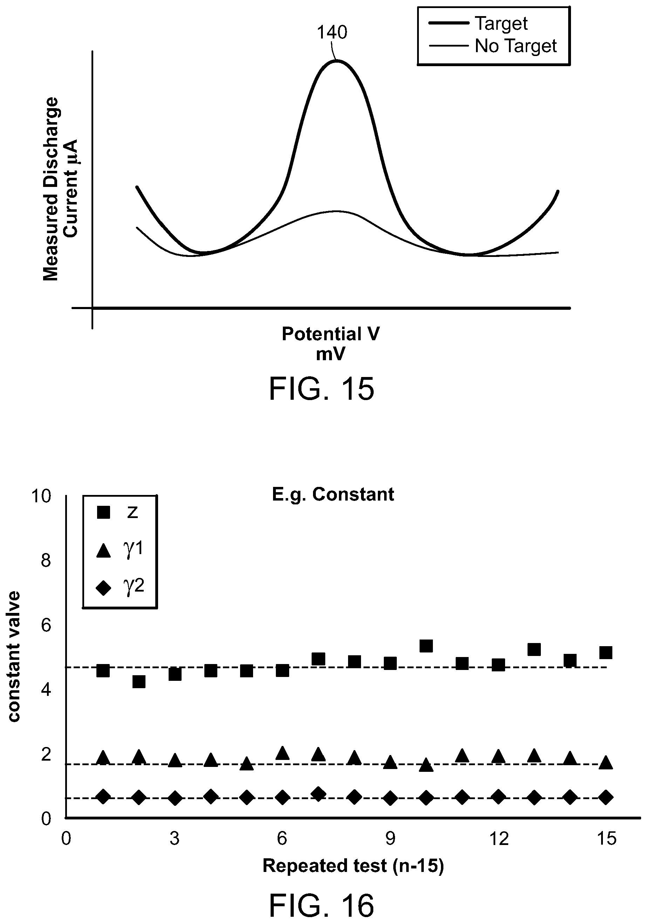

The electrodes 310-318 are operably linked by PCB vias 334 to contact points 320-328 on the bottom surface 32 of the test strip 3, as shown in FIG. 11. (In the embodiment depicted in Figs. 9 to 11, contact points 331 and 332 are currently unused.) The vias should be filled with metal or designed with such a small diameter such that no sample can leak through. When the contact points 320-332 of the test strip 3 make contact with corresponding pins 52 on a vertical connector 51 of a reader device 5, a potentiostatic circuit is formed, such that electrodes 310-318 become operable to be activated and sampled. For instance, using SWV, the faradic discharge resulting from conformational change of the redox-labeled aptamer can be sampled, whereby a peak current 140 can be acquired and converted to digital signal. FIG. 15 is a graphical visualization of one such SWV output, showing the clear peak current 140 in the presence of target (compared to no clear peak in the target's absence). Although the embodiments shown and described herein are "signal on" aptamers whereby the aptamers fold closer to the electrode surface, "signal off" aptamers that unfold and move away from the electrode surface, thereby causing an SWV minimum current, may also be used without departing from the spirit of the invention.

In the embodiment depicted in Figs. 9 to 11, there is a short between contact points 329-330 that acts as a short circuit switch so that once a test strip contacts the pins 52 of vertical connector 51, the switch is closed and a device controller on the circuit board 54 indicates that the test can continue. The reader device 5 incorporates standard electronics known in the art to sample the faradaic discharge and process signal into data that may be sent by Bluetooth or other communication method for further processing, interpretation, or storage.

The test strip does not require any amplification steps to increase binding induced signals to a detectable level. It does not require the use of an additional label ligand separate to the binding ligand because the redox-active molecule is covalently linked to the binding aptamer. It does not require any localisation methods to draw targets out of the main body of the test sample and into the detection region of the aptamers because natural diffusion of targets is sufficient. There are no additional reagents required to mix or prepare the biosensor as is often required in antibody-based sensors. Unlike antibody-based colorimetric platforms where once the colour change occurs, the sensor cannot be reused, the test strip is reversible and may be used in continuous sensing applications. In addition, aptamers typically have a higher specificity to targets than antibodies, meaning the aptamer will only bind to the desired target and it is less affected by molecules with similar structures. This means that there are no additional filtration steps required to remove other molecules in the test sample that could interfere with the biosensor and produce false positive signals. There is also significantly less 'background noise' in the signal because signal change only occurs due to aptamer binding and folding. Impedance sensors and FET-based sensors measure changes in the surface properties of their biosensors such as resistance. Such types of electrochemical sensors are greatly affected by molecules such as proteins non-specifically adsorbing onto the sensor surface and thus face significant additional challenges to reach commercialisation.

Typically an aptamer modified electrochemical test strip must be stored in a wet-state or if kept in a dry state it must be used within a few hours before degradation occurs. Furthermore, the metallic substrate of the electrodes is susceptible to oxidation, in which atmospheric oxygen molecules interact with the metallic surface resulting in spontaneous redox reactions, which can affect accuracy and disrupt the spatial orientation of the aptamers or break their covalent bonds. Therefore, a barrier layer 3141 of agarose hydrogel is applied over the aptamer layer 3131 of the electrodes on the top surface of the test strip 3, said layer forming a physical barrier between the aptamers and oxidants in the atmosphere. The barrier layer is a gel so it is physically stable and does not dissolve, and moreover is porous thereby allowing only smaller molecules through to the aptamer layer 3131 of the sensor during use.

The hydrogel may be formed by mixing 0.016g agarose powder with distilled water in a ratio of approximately 0.016g agarose powder to 4mL of water, heating this mixture until the until the mixture becomes clear, after which 1mL dimethylformamide is added (DMF) to the mixture. These ratios are scalable to vary the amount of hydrogel. The mixture should be pipetted directly onto aptamer immobilised electrodes while it remains in a warm temperature state. The electrodes should be incubated at room temperature for 8 hours. Results show significant increase in stability with the hydrogel layer, allowing the test strips to be stored at a wide range of temperatures for many months while still producing reliable and accurate results. Thus, the test strips have excellent environmental stability and do not require refrigeration as do antibody-based biosensors.

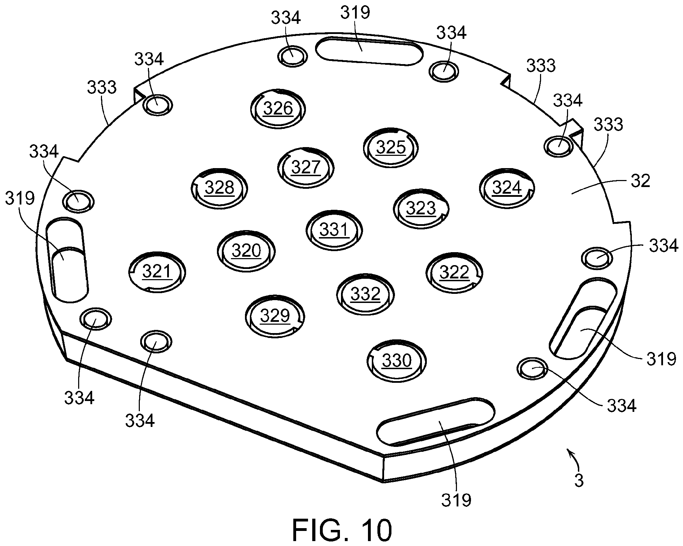

Referring to Figs 9 and 10, in the circular test strip embodiment described throughout, the test strip also contains four insertion tool slots 319 that facilitate rapid assembly into the test cartridge 1. The circular test strip 3 has small cut-aways 333 that match the profile of the test strip interface 12 thereby guiding the test strip 3 into the test cartridge 1. The test strip 3 is rotated causing the test strip to lock into place.

Waste-cartridges can be posted by a user in sealed packaging to a processing centre for waste-cartridge to be properly cleaned and processed for recycling. After appropriate cleaning, the electrode material and biochemistry can be reapplied to a PCB biosensor platform, recalibrated and re-housed in a new cartridge for future use.

III. Calibration and Use

As discussed in the Background, the output signal of aptamer sensors can vary significantly from sensor to sensor, causing most existing sensors to require a cumbersome control or blank test in order to account for this variation. This significantly increases costs of production of aptamer sensors and is impossible for sensors designed for in situ or single test disposable applications. Therefore, when pulsed voltammetry electrochemical methods are used, the test cartridge 1 may be automatically calibrated using a novel Self-Parative Calibration Method. At the time of use, the Self-Parative Calibration Method is performed, such that the target concentration of the test sample can be correctly and accurately determined without the need for testing a separate control/blank test sample.

In the case of bioreceptor with reversible binding with stoichiometry 1:1, the formula for determining analyte concentration [T] can be written as:

$$\text{Equation 1: } [T] = Kd \frac{\frac{i_1}{i_2}\frac{(\gamma_2-1)}{(\gamma_2-\gamma_1)*z}+\frac{1-\gamma_1}{\gamma_2-\gamma_1}-1}{1-\gamma_2*(\frac{i_1}{i_2}*\frac{\gamma_2-1}{(z\gamma_2-z\gamma_1)}+\frac{1-\gamma_1}{\gamma_2-\gamma_1})}$$

wherein $Kd$ is the known measurement of the binding affinity of the aptamer and the target, known as the dissociation constant, $\gamma_1$ is the ratio of $\frac{i_{max1}}{i_{min1}}$, $\gamma_2$ is the ratio of $\frac{i_{max2}}{i_{min2}}$, and $z$ is the ratio of $\frac{i_{min1}}{i_{min2}}$, wherein $i_x$ represents pulse voltammetry peak height at frequency $x$, and $minx$ and $maxx$ represent SWV peak height at frequency x without and with target binding, respectively.

This formula was derived after extensive experimentation showed that $i_{min1}/i_{min2}$, denoted as z, as well as $\frac{i_1}{i_2}$, are constant for any two frequencies across all concentrations and all test strips of the same design, as shown in Tables 1 and 2.

**Table 1 The constant of z (In blank milk)**

| Electrodes | Test strip1 | Test strip2 | Test strip3 | Cross test-strips | | |
|---|---|---|---|---|---|---|
| WE1 | 4482 | 4.206 | 4.236 | Average | SD | RSD |
| WE2 | 4134 | 4.301 | 4.332 | 4.301 | 0.036 | 0.80% |
| WE3 | 4.284 | 4.063 | 4.525 | Cross all WE | | |
| WE4 | 4.249 | 4.376 | 4.220 | Average | SD | RSD |
| WE5 | 4434 | 4.354 | 4.326 | 4.301 | 0.121 | 2.80% |
| Average | 4.317 | 4.260 | 4.328 | | | |
| SD | 0.127 | 0.115 | 0.108 | | | |
| RSD | 2.93% | 2.69% | 2.51% | | | |

**Table 2 The variable of i1/i2 at given concentration (78uM tobramycin in milk)**

| Electrodes | Test strip1 | Test strip2 | Test strip3 | Cross test-strips | | |
|---|---|---|---|---|---|---|
| WE1 | 5663 | 4.925 | 5.431 | Average | SD | RSD |
| WE2 | 5.654 | 5.116 | 5.622 | 5.190 | 0.203 | 3.92% |
| WE3 | 5.240 | 4.765 | 5.120 | Cross all WE | | |
| WE4 | 4.892 | 5.105 | 5.082 | Average | SD | RSD |
| WE5 | 5.167 | 4.868 | 5.199 | 5.190 | 0.288 | 5.54% |
| Average | 5.323 | 4.956 | 5.291 | | | |
| SD | 0.297 | 0.136 | 0.205 | | | |
| RSD | 5.58% | 2.75% | 3.88% | | | |

Furthermore, the ratio of $i_{max1}/i_{min1}$ and $i_{max2}/i_{min2}$ are constant for any two frequencies across all concentrations and all test strips of the same design. These results are also shown in FIG. 15.

The two frequencies, along with the disassociation constant $Kd$, $\gamma_1$, $\gamma_2$ and $z$, may be determined during an initial testing stage of the sensor. The two frequencies are any two frequencies that show signal change in the presence of the analyte, and preferably two frequencies that show maximum amount of signal change. Any two frequencies can be used so long as the peak heights for a target concentration are not the same for the two frequencies. The constants and information about the selected frequencies may then be stored in a database containing an identification of each sensor and its associated frequencies and constants. At the point of use, the working electrode(s) of the sensor need only be sampled at the two frequencies, and these values input to Equation 1 to determine an accurate value for the concentration of analyte.

Once the $i_1$ and $i_2$ have been determined, the calculation of analyte concentration *[T]* may be performed by any computer, locally on the reader device 5, by remote computer 8 or computers connected via wire or wireless communicative link to the reader device, or combinations of the foregoing, using standard computing methods. Similarly, the constants associated with each aptamer may be stored locally or on remote computer or computers. A user device 7 such as a smartphone, tablet, personal computer or the like may also be connected to remote computer 8 and/or reader device 5 in order to activate the test, receive results of the test, and other user interfacing functions. As such, a method of determining an analyte concentration using test cartridge 1 is as follows:

Step 1: The test cartridge 1 is attached to the reader device 5 via vertical connector 51, whereby connection points 320-332 make contact with pins 52. The contact between connection points 330-332 and pins 52 form an operable linkage whereby the reader device is operable to activate the potentiostatic circuit formed between the counter, reference and working electrodes.

Step 2: Identifying information about the test cartridge is transmitted and information such as expiry date and test settings is retrieved for each working electrode. Test settings may include such settings as test type, the two frequencies at which the Self-Parative Calibration Method is to be performed, and the constants associated with the working electrode.

Step 3: After an indication from the user to begin the test is received, a designated time sufficient for the aptamer to bind to target, preferably 1 minute, is allowed to pass before the potentiostat is activated. After the waiting period, the first pulsed voltammetry test is performed for the working electrode pursuant to the test settings for that working electrode. In particular, potential is applied across the counter, reference and the working electrode at a first frequency. During the test, the redox-active molecule discharge current is sampled at the working electrode. Further signal processing such as noise removal and data smoothing may be applied, and identification of peak height is determined and converted by ADC converter 540 to first electrical current value $i_1$.

Step 4: The second test is then performed on the same working electrode at the second frequency identified in the test settings for that working electrode, until second electrical current value $i_2$ has been obtained.

Step 5: $i_1$ and $i_2$ are used to calculate analyte concentration *[T]* using Equation 1.


IV. Applications of Use

In consumer applications, the resulting biomarker measurements can be sent by the reader device to a user on their personal computing device and displayed in a software application. In some personal health applications a software application may also give advice to the user to optimize the tested parameters, with advice ranging from dietary, exercise or a specific expert's advice. The device and the application may communicate with a remote server to manage and store all results information. In some non-consumer applications the reader device may independently display test result information and advice to the user and communicates with a remote server without the need for a separate personal computing device.

In consumer health applications, this system allows users to monitor their health at the molecular level regularly from anywhere so there is no need to go to the hospital, clinic or testing facilities for tests. Based on test results the user can optimize their behavior to live a healthier life, allowing more personalized healthcare from the comfort of one's own home. Through measuring specific biomarkers in blood, saliva and other bodily fluids, users can achieve a healthier life, and reduce the risk of the onset of chronic conditions, influencing metabolism, weight and energy. This brings down the prohibitive costs associated with personalized healthcare, offering a cost-effective opportunity for users to test from home.

As an example of the consumer health application, this invention provides people at higher risk of heart disease, diabetes and many other chronic conditions with the ability to regularly and instantly access their own molecular health information to positively influence their own daily diet and living style. Currently people at higher risk of heart disease, diabetes and many other chronic conditions can only obtain this information by consulting doctors and carrying out a detailed blood and body fluid analysis in the hospital. This process takes a long time, therefore discouraging regular testing and demotivating people to change their diet. The reassurance people at higher risk of heart disease, diabetes and many other chronic conditions would obtain through instant body fluid testing would give them continuous support to make positive lifestyle changes to manage this risk more effectively.

As an example of the emergency and medical application, this invention provides health professionals such as doctors with the ability to regularly and instantly access their patient's molecular health information to be better able to give immediate medical advice and services. Currently doctors in hospitals and local practices are required to collect fluid samples, deliver samples to a laboratory, and await test result information to be communicated back to them before they may provide medical advice and services. This process takes a long time in some cases up to 4 hours, resulting in the condition of the patient deteriorating and the requirement of the delivery of same samples such as blood, exposes them to long wait times and unaccounted temperature changes which can negatively affect the sample quality and affect laboratory analysis which produces more risk of errors in test results. Health professionals are in a position to provide faster advice and services to a patient without the concern of sample deterioration due to the transportation of test samples to a laboratory.

As an example of the food and drinking fluid inspection application, this invention provides parents of prematurely born babies consuming breastmilk and other liquid foods such as powdered milk, with the ability to regularly and instantly access their baby's food and drinking fluids molecular nutrition information such as Vitamin B 12, IgA and Vitamin D and check for contaminants such as BPA, melamine and heavy metals such as Lead, to be better able to manage their baby's diet and health. Currently parents of prematurely born babies are required to visit hospitals for analysis of breastmilk content to ensure that premature babies that are typically in a very unstable health condition, are receiving sufficient nutrition and not exposed to contaminants that can harm them. Parents are also reliant upon accurate nutritional information printed on product packaging but do not have the ability to assess whether the purchased product is from a legitimate source, has been tampered with in the product supply chain or displays misleading information within the printed information. Parents are in a position to receive fast and accurate molecular analysis of food and drinking fluids before they give them to their baby and can be reassured that their baby's nutritional intake is properly managed.

As an example of the drug detection application, this invention provides police and security services with the ability to instantly access the molecular content of individuals under-investigation for consuming prohibited substances such as cocaine, cannabinoids and amphetamines. Presently police and security services are required to collect fluid samples, deliver samples to a laboratory, and await test result information to be communicated back to them before they pass judgement on the individual under-investigation. This process takes a long time in some cases requiring the individual under-investigation to remain under custody of the police and security services before test results are received. The requirement of the delivery of same samples such as saliva, exposes them to possible tampering, replacement and manipulation which would result in an erroneous test result. Police and security services are in a position to conduct faster inspection services without the concern of sample tampering due to the transportation of test samples to a laboratory.

As an example of the environmental inspection application, this invention provides governmental and non-governmental organisations monitoring the condition of rivers and waterways, with the ability to regularly and instantly analyse water molecular information for contaminants such as herbicides such as glyphosate and pesticides and chemical dumping, to be better able to manage the conditions of rivers and waterways. Currently inspection services are required to periodically visit rivers and waterways for analysis of containment content to ensure that they remain in a clean and non-dangerous condition. Inspection services do not have the resources to more frequently monitor all rivers and waterways and are reliant on periodic visits that result in incomplete analysis of river and waterway conditions. By distributing the invention to members of the public who live more frequently use the rivers and waterways, members of the public are able to conduct frequent testing of the rivers and waterways and automatically communicate test result data to a server accessed by the inspection services, providing more information to the inspection services. The inspections services are in a position to receive frequent and accurate molecular analysis of rivers and waterways and thus better able to manage the condition of rivers and waterways under their jurisdiction.

## Claims

1. A test strip (3) for reporting a plurality of analyte concentrations of a sample, the test strip comprising:

> a top surface (31);
> a bottom surface (32),
> wherein on the top surface (31) there are a counter electrode (311), a reference electrode (310) and a plurality of working electrodes (312-318), each working electrode of the plurality of working electrodes being a PCB electrode comprised of a conductive layer (3121) and an aptamer layer (3131) comprised of aptamers (3122) that are immobilized to the conductive layer;
> wherein each aptamer (3122) is modified with a redox-active molecule (3123) selected such that the aptamer exhibits conformation change in the presence of an analyte, namely changing proximity of the aptamers to the conductive layer (3121); and
> wherein on the bottom surface (932) there is a plurality of contact points (320-331), each contact point operably linked with the counter electrode (311), reference electrode (310), and plurality of working electrodes (312-318) of the top surface (31).

**2.** The test strip of Claim 1, wherein each working electrode (312-318) is associated with a first frequency, a second frequency, a dissociation constant *Kd,* and constants $\gamma_1$, $\gamma_2$, and *z* wherein the analyte concentration at each working electrode is determined by

$$[T] = Kd \frac{\dfrac{i_1}{i_2}\dfrac{(\gamma_2 - 1)}{(\gamma_2 - \gamma_1)*z} + \dfrac{1 - \gamma_1}{\gamma_2 - \gamma_1} - 1}{1 - \gamma_2 * (\dfrac{i_1}{i_2} * \dfrac{\gamma_2 - 1}{(z\gamma_2 - z\gamma_1)} + \dfrac{1 - \gamma_1}{\gamma_2 - \gamma_1})}$$

wherein $i_1$ is a first electrical current value received from the working electrode corresponding to said analyte at the first frequency and $i_2$ is a second electrical current value received from the working electrode corresponding to said analyte at the second frequency.

**3.** The test strip of Claim 1 or 2, wherein the PCB electrode further comprises a barrier layer separate from and covering the aptamer layer completely, the barrier layer comprised of agarose powder mixed with water and dimethylformamide.

**4.** An integrated test cartridge for determining one or more analyte concentrations of a sample comprising a test strip (3) as claimed in claim 1, 2 or 3 and:

a sample collector (2) comprising a receptacle with a top opening for receiving the sample and a base in direct fluid connection with the sample collector;
a test strip interface (12) configured to accept said test strip (3) in watertight connection with the base of the sample collector (2)
such that the test strip interface (12) lies directly beneath the base of the sample collector (2), said base having an opening (21) whereby the plurality of electrodes (310-318) of the top surface (31) of the test strip (3) are simultaneously in direct fluid connection with the sample collector (2); and
a reader device interface (4) configured to attach to a vertical connector (51) on a reader device (5) comprising a circuit board (54) in operable connection with the vertical connector,
wherein the reader device interface (4) is located under the sample collector (2) and surrounding the test strip interface (12) whereby the plurality of contact points (320-331) of the bottom surface (32) of the test strip (3) become operably linked to the vertical connector (51) when said reader device interface (4) is attached to the vertical connector.

**5.** The integrated test cartridge of Claim 4, wherein the test strip interface (12) comprises an O-ring (25) beneath the base of the sample collector (2) and surrounding the opening (21) of the base of the sample collector, and a plurality of insertion clips (121) located beneath the base of the sample collector and beneath the O-ring such that the insertion clips secure the test strip (3) against the O-ring, and
wherein the test strip (3) is substantially in the shape of a disc and comprises cutaways (333) complementary to the insertion clips (121), the insertion clips (121) of the test strip (3) being configured to fit into the cutaways (333) to enable the test strip to be secured against the O-ring (25) by rotation of the test strip relative to the base of the sample collector (2).

**Patentansprüche**

**1.** Teststreifen (3) zum Berichten einer Vielzahl von Analytkonzentrationen einer Probe, wobei der Teststreifen umfasst:

eine obere Fläche (31);
eine untere Fläche (32),
wobei auf der oberen Fläche (31) eine Gegenelektrode (311), eine Referenzelektrode (310) und eine Vielzahl von Arbeitselektroden (312-318) vorliegen, wobei jede Arbeitselektrode von der Vielzahl von Arbeitselektroden eine PCB-Elektrode ist, die aus einer leitfähigen Schicht (3121) und eine Aptamerschicht (3131) besteht, die aus Aptameren (3122) besteht, die an der leitfähigen Schicht immobilisiert sind;
wobei jedes Aptamer (3122) mit einem redoxaktiven Molekül (3123) modifiziert ist, das derart ausgewählt ist, dass das Aptamer eine Konformationsänderung in Gegenwart eines Analyten zeigt, nämlich eine Änderung der Nähe der Aptamere zu der leitfähigen Schicht (3121); und
wobei auf der unteren Fläche (932) eine Vielzahl von Kontaktstellen (320-331) vorliegt, wobei jede Kontaktstelle funktionsfähig mit der Gegenelektrode (311), der Referenzelektrode (310) und der Vielzahl von Arbeitselektroden (312-318) der oberen Fläche (31) verknüpft ist.

2. Teststreifen nach Anspruch 1, wobei jede Arbeitselektrode (312-318) mit einer ersten Frequenz, einer zweiten Frequenz, einer Dissoziationskonstante $Kd$ und Konstanten $\gamma_1$, $\gamma_2$ und assoziiert ist, wobei die Analytkonzentration an jeder Arbeitselektrode bestimmt wird durch

$$[T] = Kd \frac{\dfrac{i_1}{i_2}\dfrac{(\gamma_2 - 1)}{(\gamma_2 - \gamma_1) * z} + \dfrac{1 - \gamma_1}{\gamma_2 - \gamma_1} - 1}{1 - \gamma_2 * \left(\dfrac{i_1}{i_2} * \dfrac{\gamma_2 - 1}{(z\gamma_2 - z\gamma_1)} + \dfrac{1 - \gamma_1}{\gamma_2 - \gamma_1}\right)}$$

wobei $i_1$ ein erster elektrischer Stromwert ist, der von der Arbeitselektrode empfangen wird und dem Analyten bei der ersten Frequenz entspricht, und $i_2$ ein zweiter elektrischer Stromwert ist, der von der Arbeitselektrode empfangen wird und dem Analyten bei der zweiten Frequenz entspricht.

3. Teststreifen nach Anspruch 1 oder 2, wobei die PCB-Elektrode weiterhin eine Barriereschicht umfasst, die von der Aptamerschicht separat ist und diese vollständig bedeckt, wobei die Barriereschicht aus Agarosepulver besteht, das mit Wasser und Dimethylformamid gemischt ist.

4. Integrierte Testkartusche zum Bestimmen einer oder mehrerer Analytkonzentrationen einer Probe, umfassend einen Teststreifen (3) nach Anspruch 1, 2 oder 3 und

einen Probensammler (2), umfassend ein Behältnis mit einer oberen Öffnung zum Aufnehmen der Probe und eine Basis in direkter Fluidverbindung mit dem Probensammler;
eine Teststreifenschnittstelle (12), die dazu konfiguriert ist, den Teststreifen (3) in einer wasserdichten Verbindung mit der Basis des Probensammlers (2) entgegenzunehmen,
so dass die Teststreifenschnittstelle (12) direkt unter der Basis des Probensammlers (2) liegt, wobei die Basis eine Öffnung (21) aufweist, wodurch die Vielzahl von Elektroden (310-318) der oberen Fläche (31) des Teststreifens (3) gleichzeitig in direkter Fluidverbindung mit dem Probensammler (2) ist; und
eine Lesegeräteschnittstelle (4), die dazu konfiguriert ist, sich an einen vertikalen Verbinder (51) auf einem Lesegerät (5) anzubringen, das eine Leiterplatte (54) in funktionsfähiger Verbindung mit dem vertikalen Verbinder umfasst,
wobei die Lesegeräteschnittstelle (4) sich unter dem Probensammler (2) befindet und die Teststreifenschnittstelle (12) umgibt, wodurch die Vielzahl von Kontaktstellen (320-331) der unteren Fläche (32) des Teststreifens (3) funktionsfähig mit dem vertikalen Verbinder (51) verknüpft wird, wenn die Lesegeräteschnittstelle (4) an dem vertikalen Verbinder angebracht wird.

5. Integrierte Testkartusche nach Anspruch 4, wobei die Teststreifenschnittstelle (12) einen O-Ring (25) unter der Basis des Probensammlers (2), der die Öffnung (21) der Basis des Probensammlers umgibt, und eine Vielzahl von Einschubklammern (121), die sich unter der Basis des Probensammlers und unter dem O-Ring befindet, umfasst, so dass die Einsatzklammern den Teststreifen (3) gegen den O-Ring sichern, und
wobei der Teststreifen (3) im Wesentlichen in der Form einer Scheibe ist und Ausschnitte (333) umfasst, die zu den Einschubklammern (121) komplementär sind, wobei die Einschubklammern (121) des Teststreifens (3) dazu konfiguriert sind, in die Ausschnitte (333) zu passen, um zu ermöglichen, dass der Teststreifen durch eine Drehung des Teststreifens in Bezug auf die Basis des Probensammlers (2) gegen den O-Ring (25) gesichert wird.

**Revendications**

1. Une bande de test (3) destinée à faire rapport d'une pluralité de concentrations d'analyte d'un échantillon, la bande de test comprenant :

   une surface supérieure (31) ;
   une surface inférieure (32),
   dans laquelle sur la surface supérieure (31), il y a une contre-électrode (311), une électrode de référence (310) et une pluralité d'électrodes de travail (312-318), chaque électrode de travail de la pluralité d'électrodes de travail étant une électrode de PCB composée d'une couche conductrice (3121) et d'une couche d'aptamère (3131) composée d'aptamères (3122) qui sont immobilisés sur la couche conductrice ;
   dans laquelle chaque aptamère (3122) est modifié par une molécule à activité redox (3123) sélectionnée de telle sorte que l'aptamère présente un changement de conformation en présence d'un analyte, à savoir un changement de proximité des aptamères sur la couche conductrice (3121) ; et
   dans laquelle sur la surface inférieure (932), il y a une pluralité de points de contact (320-331), chaque point de contact étant lié fonctionnellement avec la contre-électrode (311), l'électrode de référence (310) et une pluralité d'électrodes de travail (312-3 l 8) de la surface supérieure (31).

2. La bande de test selon la revendication 1, dans laquelle chaque électrode de travail (312-318) est associée à une première fréquence, une deuxième fréquence, une constante de dissociation $Kd$ et des constantes $\gamma_1$, $\gamma_2$ et $z$, dans laquelle la concentration d'analyte à chaque électrode de travail est déterminée par

   $$[T] \quad Kd \, \frac{\dfrac{i_1}{i_2}\dfrac{(\gamma_2-1)}{(\gamma_2-\gamma_1)*z}+\dfrac{1-\gamma_1}{\gamma_2-\gamma_1}-1}{1-\gamma_2*\left(\dfrac{i_1}{i_2}*\dfrac{\gamma_2-1}{(z\gamma_2-z\gamma_1)}+\dfrac{1-\gamma_1}{\gamma_2-\gamma_1}\right)}$$

   dans laquelle $i_1$ est une première valeur de courant électrique reçue de l'électrode de travail correspondant audit analyte à la première fréquence et $i_2$ est une deuxième valeur de courant électrique reçue de l'électrode de travail correspondant audit analyte à la deuxième fréquence.

3. La bande de test selon la revendication 1 ou 2, dans laquelle l'électrode de PCB comprend en outre une couche barrière distincte et recouvrant la couche d'aptamère complètement, la couche barrière composée de poudre d'agarose étant mélangée avec de l'eau et du diméthylformamide.

4. Une cartouche de test intégrée destinée à déterminer une ou plusieurs concentrations d'analyte d'un échantillon comprenant une bande de test (3) selon la revendication 1, 2 ou 3 et :

   un collecteur d'échantillon (2) comprenant un réceptacle ayant une ouverture supérieure pour recevoir l'échantillon et une base en communication fluidique directe avec le collecteur d'échantillon ;
   une interface de bande de test (12) configurée pour accepter ladite bande de test (3) en communication étanche avec la base du collecteur d'échantillon (2)
   de telle sorte que l'interface de bande de test (12) se trouve directement au-dessous de la base du collecteur d'échantillon (2), ladite base ayant une ouverture (21) grâce à quoi la pluralité d'électrodes (310-318) de la surface supérieure (31) de la bande de test (3) sont simultanément en communication fluidique directe avec le collecteur d'échantillon (2) ; et
   une interface de dispositif de lecture (4) configurée pour se fixer à un connecteur vertical (51) sur un dispositif de lecture (5) comprenant une carte de circuit (54) en communication fonctionnelle avec le connecteur vertical, dans laquelle l'interface de dispositif de lecture (4) est située sous le collecteur d'échantillon (2) et entourant l'interface de bande de test (12) grâce à quoi la pluralité de points de contact (320-331) de la surface inférieure (32) de la bande de test (3) deviennent liés fonctionnellement au connecteur vertical (51) lorsque ladite interface de dispositif de lecture (4) est fixée au connecteur vertical.

5. La cartouche de test intégrée selon la revendication 4, dans laquelle l'interface de bande de test (12) comprend un joint torique (25) au-dessous de la base du collecteur d'échantillon (2) et entourant l'ouverture (21) de la base du collecteur d'échantillon, et une pluralité d'attaches d'insertion (121) située au-dessous de la base du collecteur d'échantillon et au-dessous du joint torique de telle sorte que les attaches d'insertion assujettissent la bande de test (3) contre le joint torique, et

dans laquelle la bande de test (3) est sensiblement de la forme d'un disque et comprend des découpes (333) complémentaires des attaches d'insertion (12] ), les attaches d'insertion (121) de la bande de test (3) étant configurées pour s'introduire dans les découpes (333) pour permettre à la bande de test d'être assujettie contre le joint torique (25) par la rotation de la bande de test par rapport à la base du collecteur d'échantillon (2).

FIG. 1

FIG. 2

FIG. 3

FIG. 4

FIG. 5

FIG. 6

FIG. 7

Server 8

User Device 7

5

FIG. 8

FIG. 9

FIG. 10

334
334
334
334
314
315
316
311
334
334
310
313
317
312
318
334
334
31
334
334
334

334
334
334
326
325
324
334
334
327
323
32
328
331
322
334
334
321
320
332
329
330
334
334

FIG. 11

FIG. 12

FIG. 13

FIG. 14

FIG. 15

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Multiplex PCB-based electrochemical detection of cancer biomarkers using MLPA-barcode approach. **SANCHEZ J L ACERO et al.** BIOSENSORS AND BIOELECTRONICS. ELSEVIER SCIENCE LTD, 07 April 2016, vol. 82
- **HADI MIRZAJANI et al.** A highly sensitive and specific capacitive aptasensor for rapid and label-free trace analysis of Bisphenol A (BPA) in canned foods. *BIOSENSORS AND BIOELECTRONICS,* 01 March 2017, vol. 89
- **LEI WANG et al.** An Electrochemical Aptasensor Using Coaxial Capillary with Magnetic Nanoparticle, Urease Catalysis and PCB Electrode for Rapid and Sensitive Detection of Escherichia coli O157:H7. *NANOTHERANOSTICS,* 01 January 2017, vol. 1 (4
- **LIU et al.** Achieving Reproducible Performance of Electrochemical, Folding Aptamer-Based Sensors on Microelectrodes: Challenges and Prospects. *Analytical Chemistry,* 2014, vol. 86, 11417-11424